# EUROPEAN PATENT APPLICATION

(11) **EP 3 263 061 A1**
(43) Date of publication of application: **03.01.2018**
(21) Application number: 16755156.3
(22) Date of filing: 03.02.2016
(51) Int. Cl.: A61B 90/00, A61B 1/00

(54) **OVERTUBE AND MEDICAL MANIPULATOR SYSTEM**

(30) Priority: 26.02.2015 JP 2015037405
(71) Applicant: Olympus Corporation, Hachioji-shi, Tokyo 192-8507 (JP)
(72) Inventor: ARUGA, Toshinao, Hachioji-shi, Tokyo 192-8507 (JP); YANAGIHARA, Masaru, Hachioji-shi, Tokyo 192-8507 (JP); KISHI, Kosuke, Hachioji-shi, Tokyo 192-8507 (JP)
(74) Representative: Schicker, Silvia
(86) International application number: PCT/JP2016/053156
(87) International publication number: WO 2016/136406

(57) **Abstract**

The present invention enables simple confirmation that an appropriate overtube has been selected, when using a manipulator via a channel in an overtube. Provided is an overtube (4) comprising: a manipulator channel (13) through which is passed a long manipulator in which a movable portion disposed at a distal end is driven by a driving portion disposed at a proximal end; and a storage unit (24) that stores identification information for the manipulator channel (13) in a readable manner from outside.

## Description

### {Technical Field}

The present invention relates to an overtube and a medical manipulator system.

### {Background Art}

In the related art, there is a known surgical system in which a manipulator for performing treatment on a diseased part inside a body is provided with a storage means for storing information about the manipulator, and when the manipulator is connected to a control circuit, the manipulator information is passed to the control circuit (for example, see Patent Literature 1).

### {Citation List}

### {Patent Literature}

{PTL 1} Japanese Unexamined Patent Application, Publication No. 2014-57854

### {Summary of Invention}

### {Technical Problem}

However, to improve the ease of insertion of a manipulator, in some cases the manipulator is used while inserted into a channel of an overtube having a channel through which the manipulator is made to pass. Manipulators of different types, depending on the treatment site and the procedure are prepared, and as for the overtube through which the manipulator is passed, it is also necessary to select one that is appropriate for the manipulator to be used.

The present invention has been conceived in light of the circumstances described above, and an object thereof is to provide an overtube and a medical manipulator system that allow easy confirmation that an appropriate overtube has been selected, when using a manipulator via a channel in an overtube.

To achieve the above-described object, the present invention provides the following solutions.

An aspect of the present invention provides an overtube comprising: a manipulator channel through which is passed a long manipulator in which a movable portion disposed at a distal end is driven by a driving portion disposed at a proximal end; and a storage unit that stores identification information for the manipulator channel in a readable manner from outside.

With this aspect, the manipulator is made to pass through the manipulator channel in the overtube whose distal end is disposed inside the body, the movable portion is made to protrude from the distal end of the overtube, and the movable portion is driven by the driving portion disposed at the proximal end of the overtube, whereby it is possible to treat a diseased part inside the body.

In this case, by reading, from the outside, the identification information of the manipulator channel, which is stored in the storage unit provided in the overtube, it is possible to determine whether the manipulator to be used in the treatment is suitable for the manipulator channel in the overtube inserted in the body, without having to take out the overtube from the body to check it.

The above-described aspect may further comprise an endoscope channel through which an endoscope is passed.

With this configuration, by reading out the identification information of the manipulator channel, the type of endoscope that is passed through the endoscope channel in the overtube can also be confirmed.

The above-described aspect may further comprise: a distal-end tubular portion in which the manipulator channel and the endoscope channel are provided; and a proximal-end tubular portion that is detachably provided at the proximal end of the distal-end tubular portion and that is provided with an extension channel that extends the manipulator channel at the proximal-end side, wherein the storage unit is provided in at least one of the distal-end tubular portion or the proximal-end tubular portion.

By doing so, the endoscope is passed through the endoscope channel in the distal-end tubular portion, the distal-end tubular portion and the proximal-end tubular portion are connected, the manipulator is passed through the manipulator channel in the distal-end tubular portion from the extension channel in the proximal-end tubular portion, and the endoscope and the movable portion are made to protrude from the distal end of the distal-end tubular portion. Accordingly, it is possible to perform treatment of the diseased part while observing the movable portion with the endoscope. By removing the proximal-end tubular portion from the distal-end tubular portion, it is possible to easily insert the endoscope into the distal-end tubular portion.

In this case, by reading, from the outside, the identification information of the manipulator channel, which is stored in the storage unit provided in at least one of the distal-end tubular portion and the proximal-end tubular portion, it is possible to determine whether the manipulator to be used in the treatment is suitable for the manipulator channel in the overtube inserted in the body, without having to take out the overtube from the body to check it.

In the above-described aspect, the storage unit may be formed of an electrical circuit provided in either one of the distal-end tubular portion or the proximal-end tubular portion; and a proximal-end section of the distal-end tubular portion or a proximal-end section of the proximal-end tubular portion may be provided with a connection terminal for an external apparatus to read out the identification information, when the distal-end tubular portion or the proximal-end tubular portion having said proximal-end section is connected to the external apparatus.

By doing so, when the distal-end tubular portion and the proximal-end tubular portion are connected, the external apparatus can electrically read out the identification information stored in the storage unit via the connection terminal provided at the proximal-end side of the proximal-end tubular portion.

In the above-described aspect, the storage unit may be formed of an electrical circuit provided in either one of the distal-end tubular portion or the proximal-end tubular portion; and a wireless transmission unit that can wirelessly read out the identification information from an external apparatus when the distal-end tubular portion and the proximal-end tubular portion are connected may be connected to the storage unit.

By doing so, when the distal-end tubular portion and the proximal-end tubular portion are connected, the external apparatus can easily read out, in a wireless manner, the identification information stored in the storage unit by using the wireless transmission unit, which is capable of readout.

In the above-described aspect, the storage unit may be provided with a distal-end storage unit that is provided in the distal-end tubular portion and that stores identification information of the manipulator channel, and a proximal-end storage unit that is provided in the proximal-end tubular portion and that stores identification information of the extension channel.

By doing to, when the distal-end tubular portion and the proximal-end tubular portion are connected, the identification information of the manipulator channel, stored in the distal-end storage unit, and the identification information of the extension channel, stored in the proximal-end storage unit, can be read out to the external apparatus. Accordingly, it is possible to determine whether each of the distal-end tubular portion and the proximal-end tubular portion, which are detachable, is suitable for the manipulator to be used.

In the above-described aspect, the distal-end storage unit and the proximal-end storage unit may be formed of electrical circuits that are connected when the distal-end tubular portion and the proximal-end section of the proximal-end tubular portion are connected; and a connection terminal for an external apparatus to read out the identification information when the proximal-end tubular portion is connected to the external apparatus may be provided in the proximal-end tubular portion.

By doing so, when the distal-end tubular portion and the proximal-end tubular portion are connected, the external apparatus can electrically read out the identification information of the manipulator channel, stored in the distal-end storage unit, and the identification information of the extension channel, stored in the proximal-end storage unit, via the connection terminal provided at the proximal-end side of the proximal-end tubular portion.

In the above-described aspect, the distal-end storage unit and the proximal-end storage unit may be formed of electrical circuits; and a wireless transmission unit that can wirelessly read out the identification information from an external apparatus when the distal-end tubular portion and the proximal-end tubular portion are connected may be connected to at least one of the distal-end storage unit or the proximal-end storage unit.

By doing so, when the distal-end tubular portion and the proximal-end tubular portion are connected, the external apparatus can easily read out, in a wireless manner, the identification information of the manipulator channel, stored in the distal-end storage unit, and the identification information of the extension channel, stored in the proximal-end storage unit, by using the wireless transmission unit, which capable of readout.

In the above-described aspect, the storage unit may store usage count information in a readable and writable manner.

By doing so, when reusing the overtube, the usage count thereof can be managed.

In the above-described aspect, a plurality of the manipulator channels may be provided.

By doing so, the types of the overtubes, in which the position of each manipulator channel that opens at the distal end face differs, can also be confirmed without having to remove the overtube, which is inserted into the body, from the body and check it.

In the above-described aspect, the distal-end tubular portion may be provided with a plurality of the manipulator channels; the proximal-end tubular portion may be provided with, in the same number as the number of manipulator channels, a plurality of the extension channels that can extend the manipulator channels, in arbitrary combinations; the distal-end storage unit may be provided in each of the manipulator channels; and the proximal-end storage unit may be provided in each of the extension channels.

By doing so, when connecting the distal-end tubular portion and the proximal-end tubular portion, once the manipulators channels and extension channels are connected in arbitrary combinations, the external apparatus can read out the identification information in the distal-end storage unit and the proximal-end storage unit, which are connected to each other. Accordingly, it can also be confirmed whether the combination of the manipulator channels and the extension channels is suitable for the manipulator to be used.

Another aspect of the present invention is a medical manipulator system comprising: any one of the above-described overtubes; the manipulator, which is inserted into the manipulator channel in the overtube; an operating portion that is operated by an operator; a control portion that controls the driving portion on the basis of an operation input that is input via the operation portion; and a readout unit that reads out information stored in the storage unit.

According to this aspect, since the information stored in the storage unit provided in the overtube is read out by the readout unit, the operator can confirm whether the manipulator to be used is suitable for the overtube without taking the overtube out of the body. In the case where it is confirmed that the manipulator is suitable, the manipulator is passed through the manipulator channel in the overtube, and by operating the operating portion, the driving portion is driven by the control portion, the movable portion disposed inside the body is operated, and thus it is possible to perform treatment on the diseased part.

Another aspect of the present invention provides a medical manipulator system comprising: the above-described overtube; the manipulator, which is inserted into the manipulator channel in the overtube; an operating portion that is operated by an operator; a control portion that controls the driving portion on the basis of an operation input that is input via the operating portion; a readout unit that reads out information stored in the storage unit; a writing unit that writes a usage count of the overtube into the storage unit; and a notifying unit that, when the usage count read out from the storage unit by the readout unit exceeds a prescribed count, issues a notification to that effect.

According to this embodiment, when the overtube is used, the usage count is written into the storage unit provided in the overtube by the writing unit, and in the next usage, the information stored in the storage unit is read out with the readout unit. When the usage count read out by the readout unit exceeds the number of treatments, a notification to that effect is given by the notifying unit; therefore, the operator can avoid using an overtube that has exceeded a prescribed usage count. Accordingly, the usage count can be managed to reuse the overtube.

### {Advantageous Effects of Invention}

An object of the present invention is to provide an overtube and a medical manipulator system with which it is possible to easily confirm that an appropriate overtube is selected, when using a manipulator via a channel in an overtube.

### {Brief Description of Drawings}

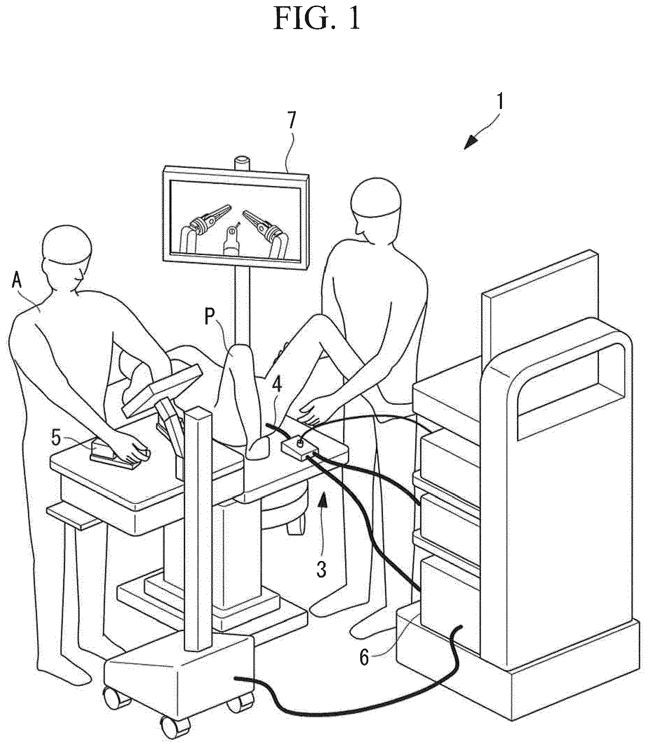

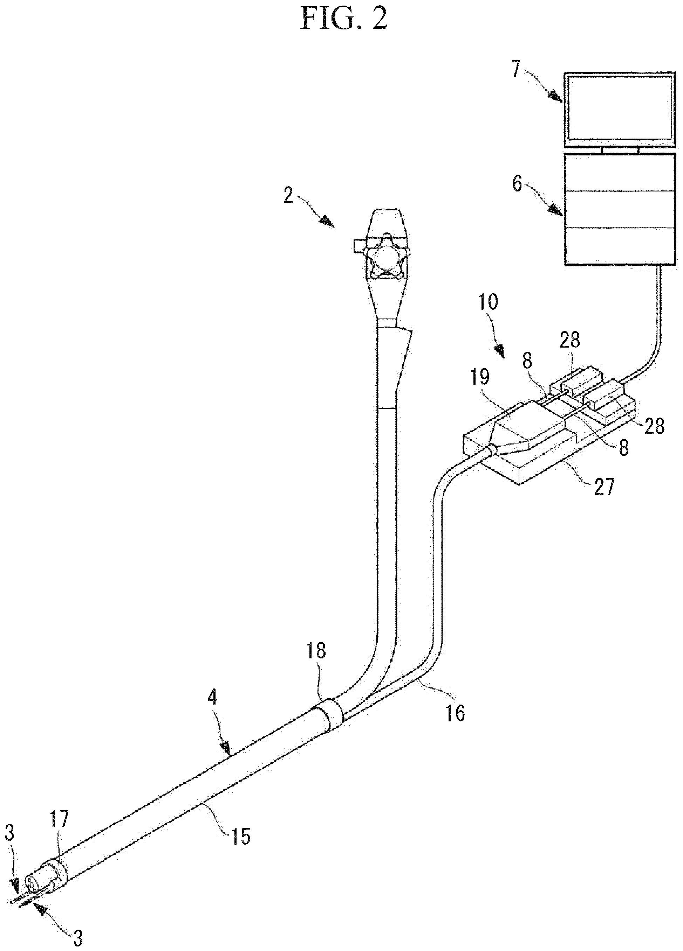

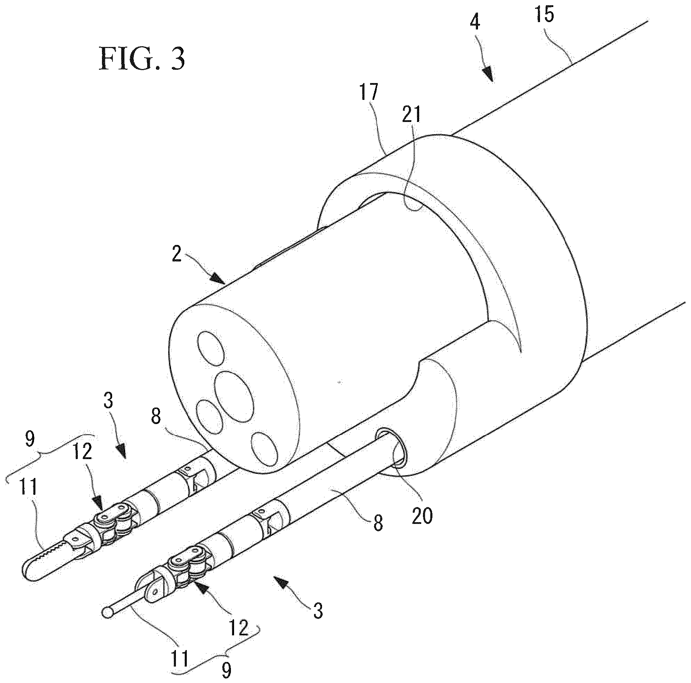

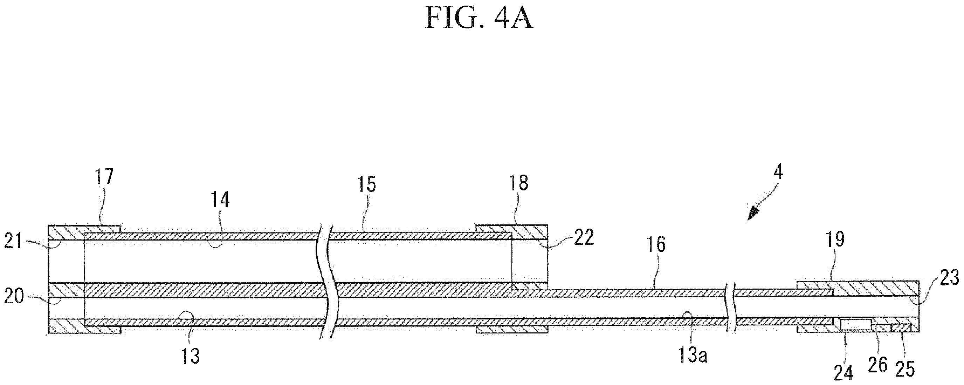

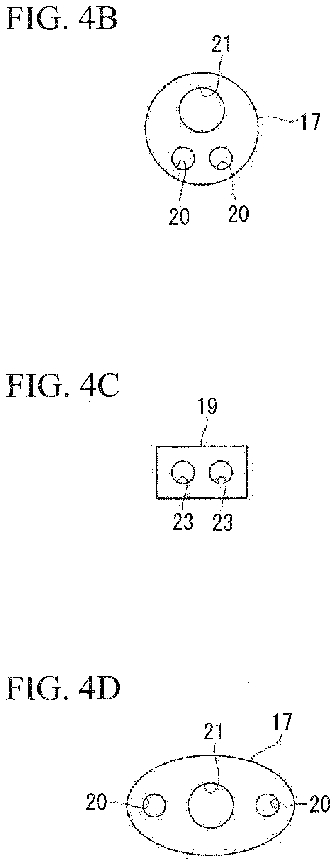

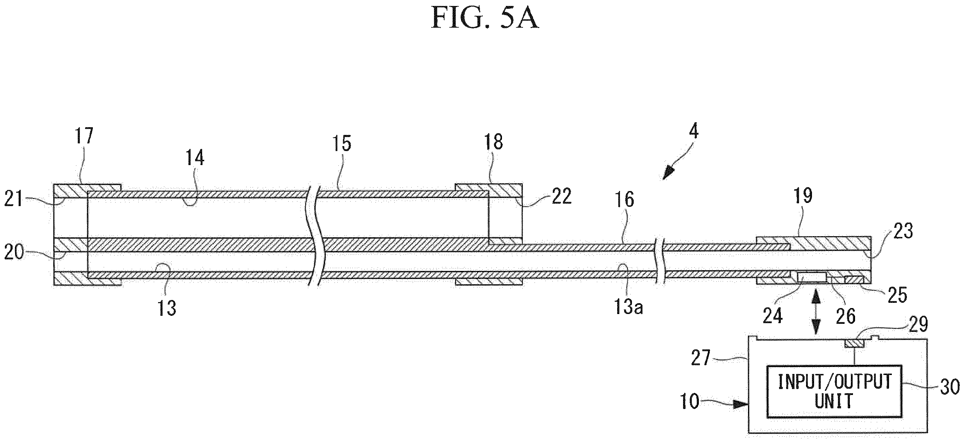

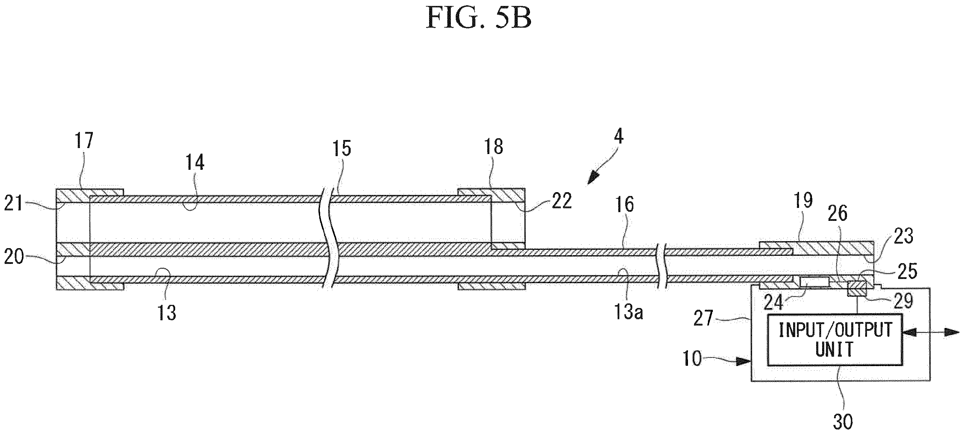

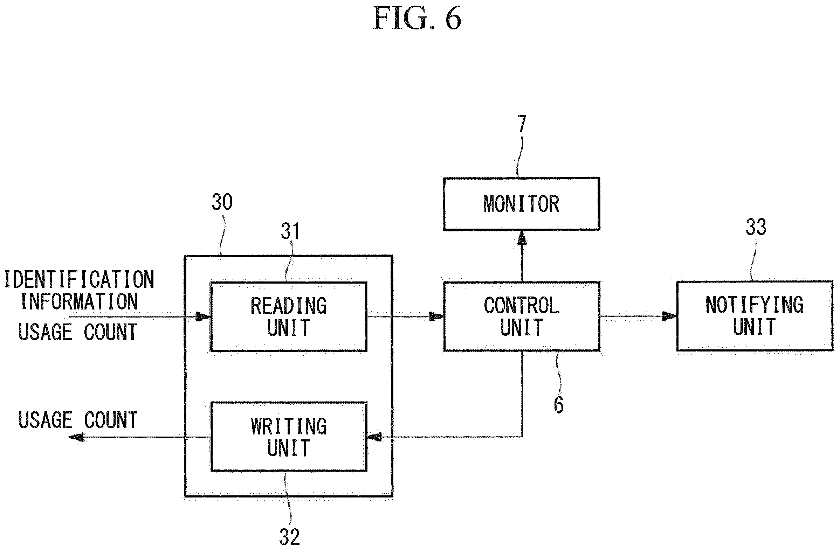

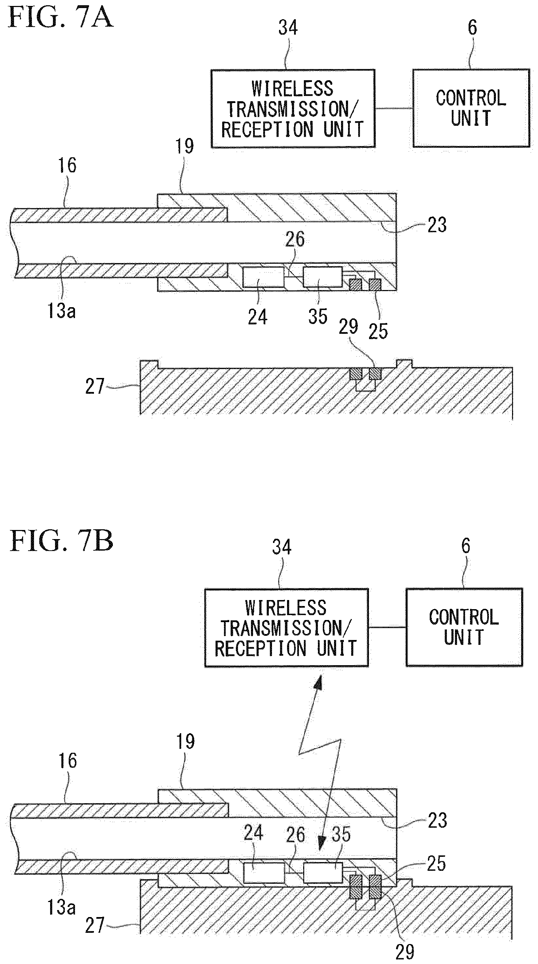

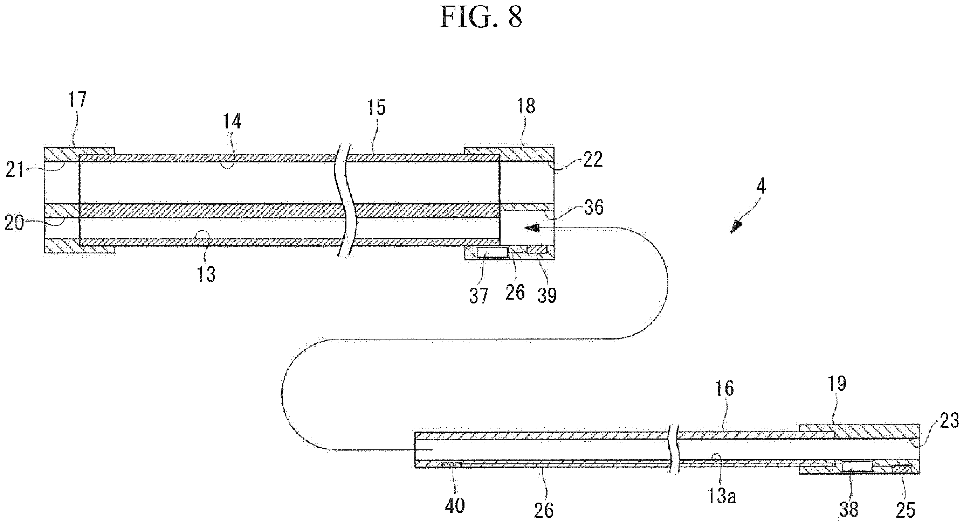

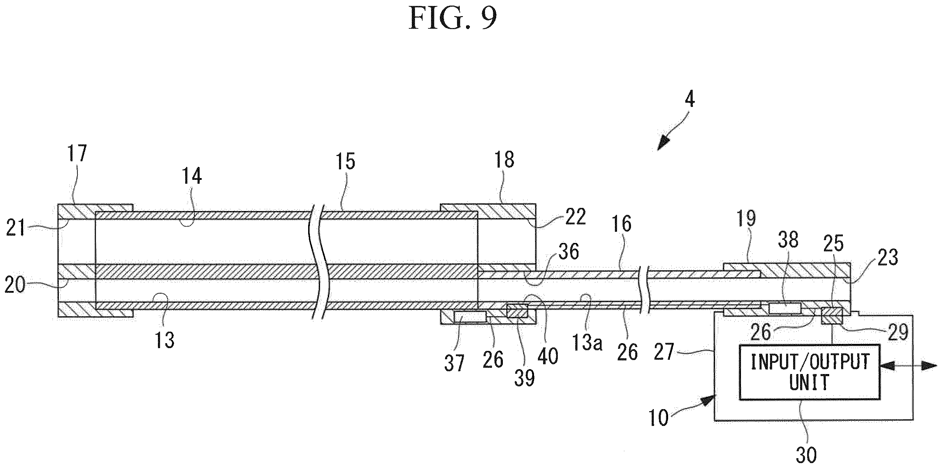

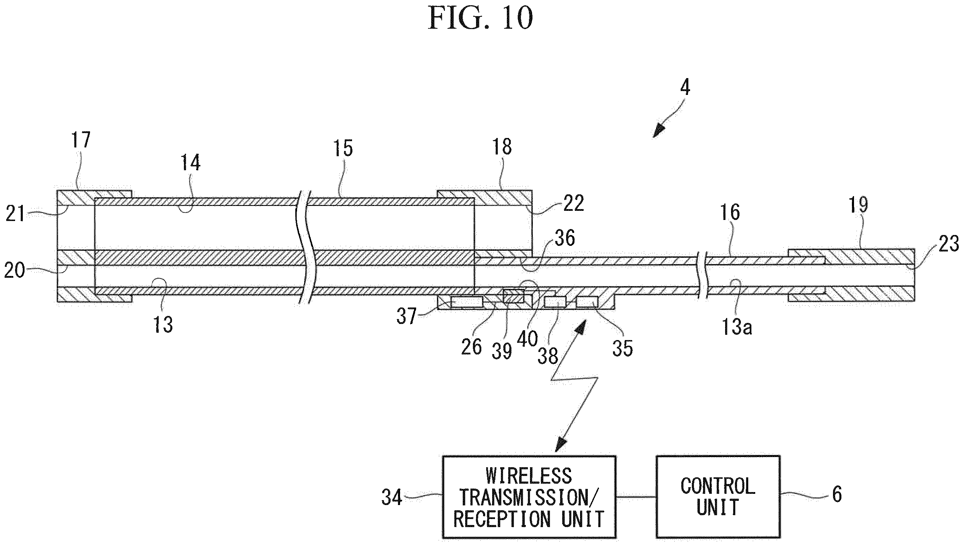

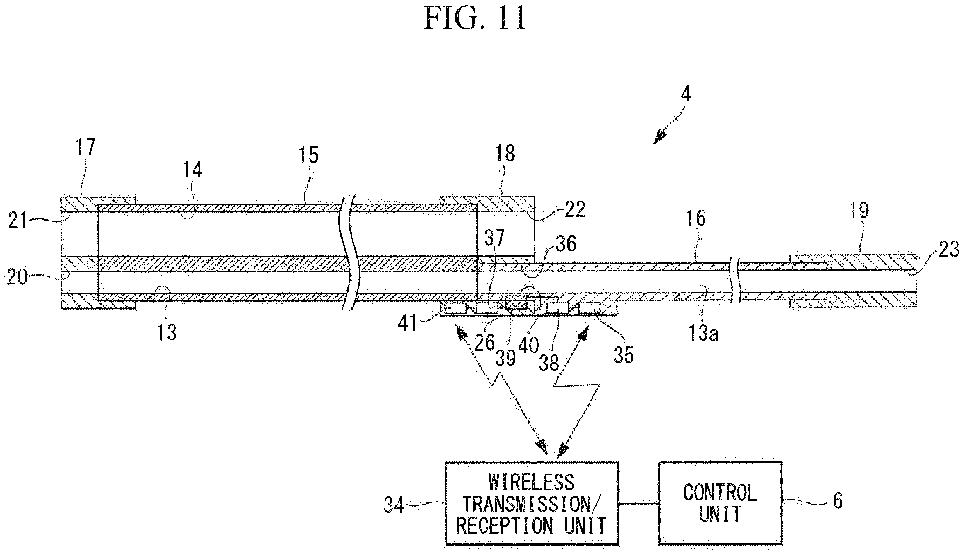

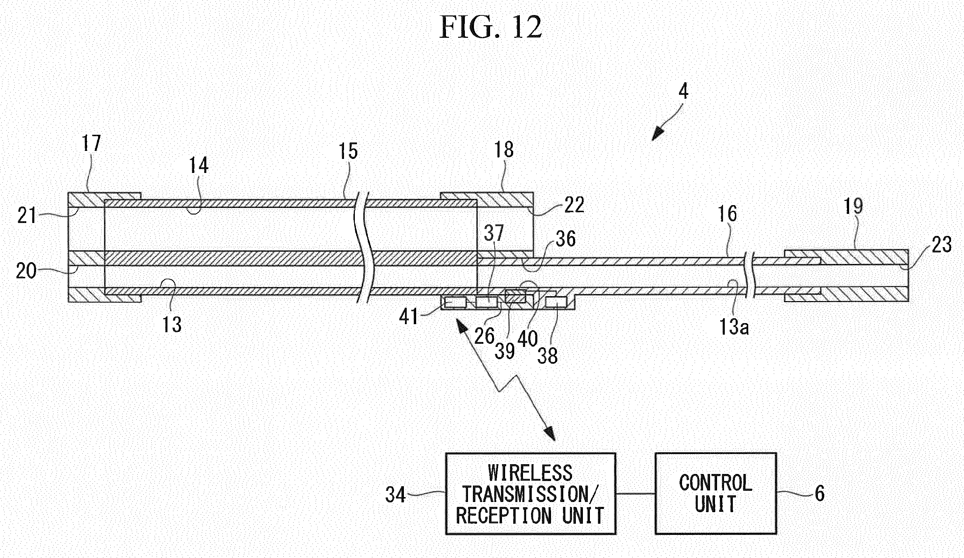

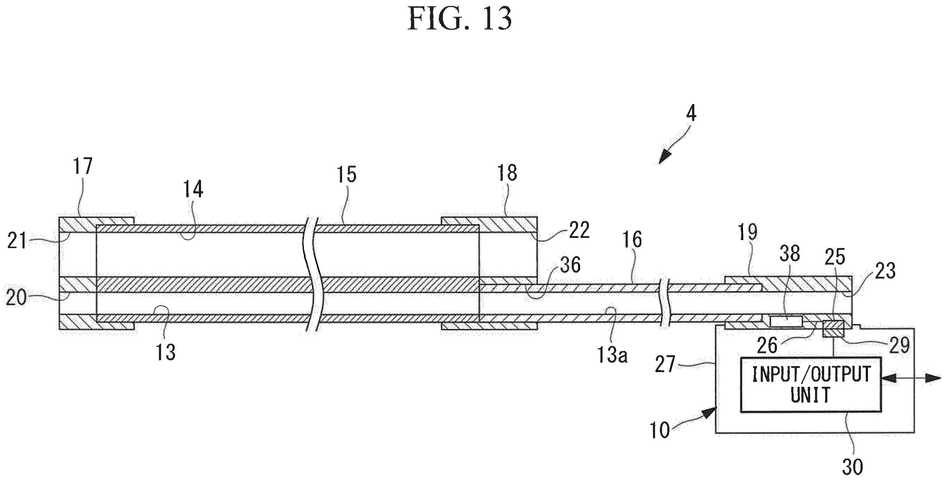

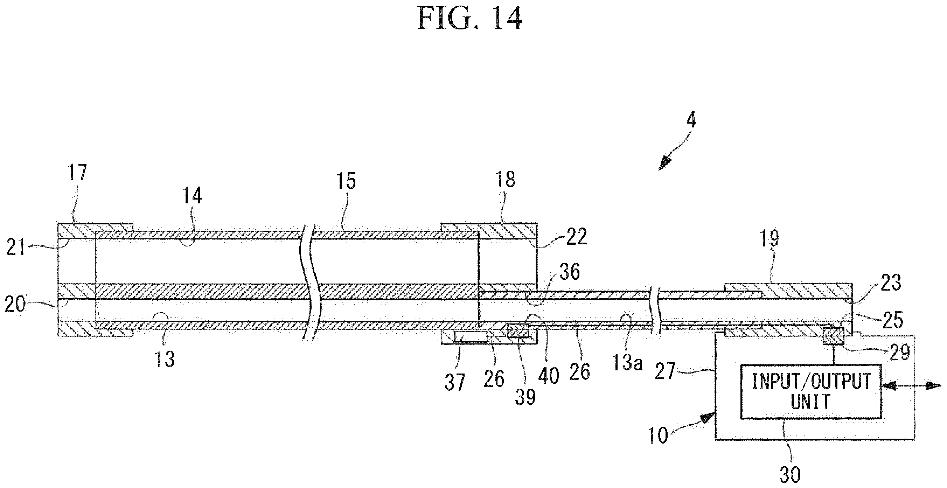

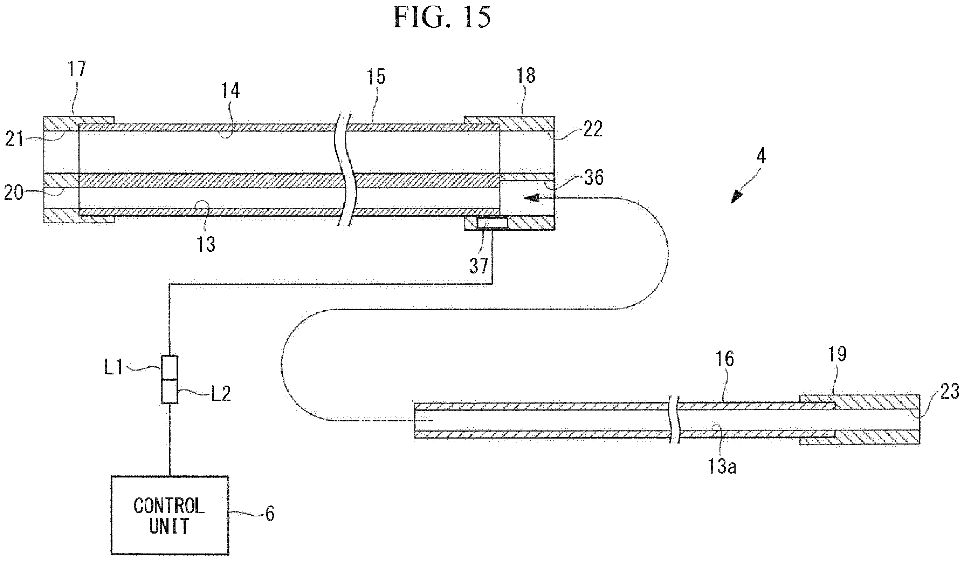

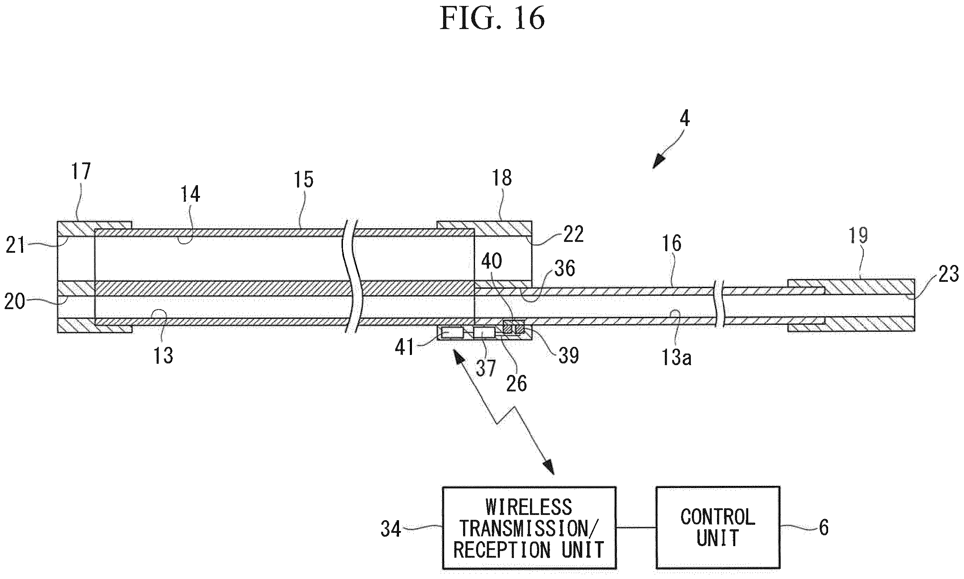

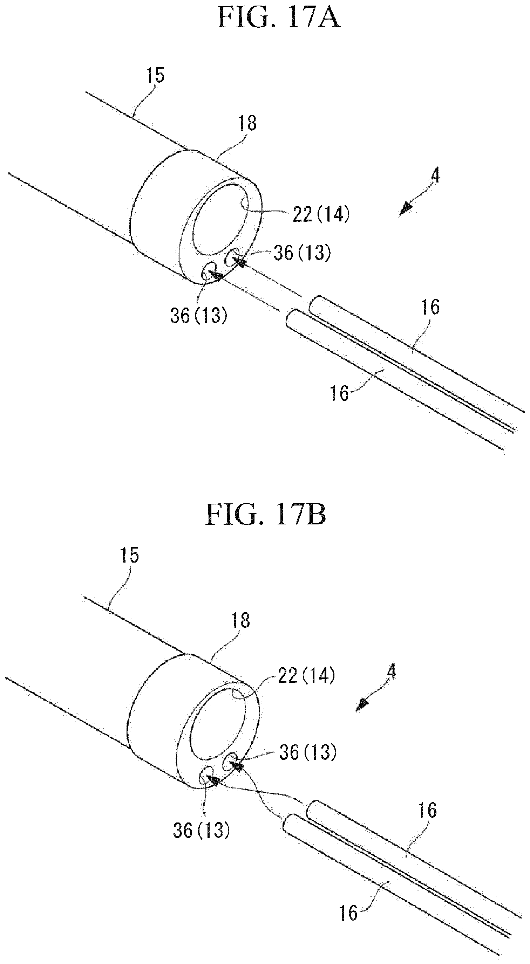

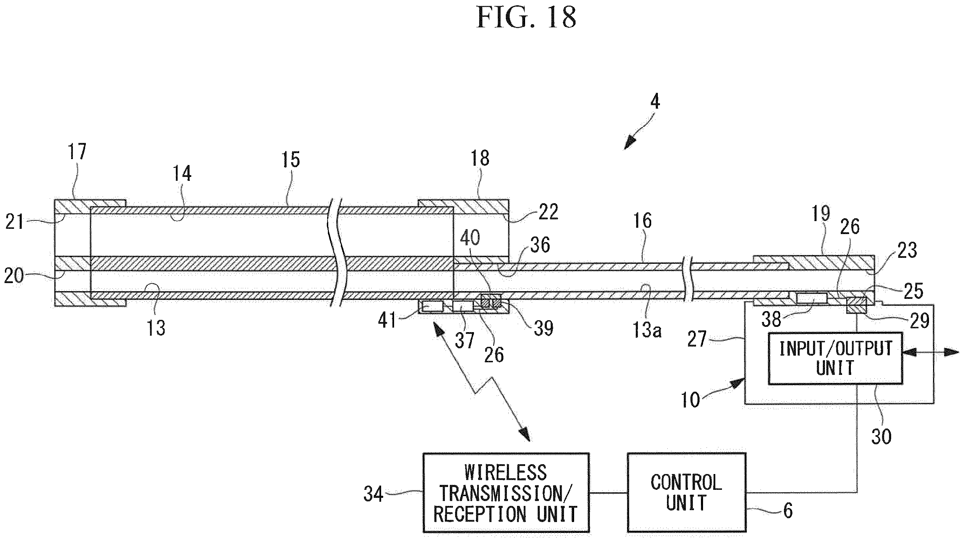

{Fig. 1} Fig. 1 is a diagram showing the overall configuration of a medical manipulator system according to an embodiment of the present invention.
{Fig. 2} Fig. 2 is a perspective view showing the medical manipulator system in Fig. 1.
{Fig. 3} Fig. 3 is a partial perspective view showing an endoscope, manipulators and the distal end of an overtube in the medical manipulator system in Fig. 2.
{Fig. 4A} Fig. 4A is a longitudinal sectional view showing the overtube according to an embodiment of the present invention, provided in the medical manipulator system in Fig. 1.
{Fig. 4B} Fig. 4B is a distal-end front elevational view showing the overtube according to the embodiment of the present invention, provided in the medical manipulator system in Fig. 1.
{Fig. 4C} Fig. 4C is a proximal-end front elevational view showing the overtube according to the embodiment of the present invention, provided in the medical manipulator system in Fig. 1.
{Fig. 4D} Fig. 4D is a distal-end front elevational view showing the overtube according to the embodiment of the present invention, provided in the medical manipulator system in Fig. 1, and is a distal-end front elevational view of a different pattern from that in Fig. 4B.
{Fig. 5A} Fig. 5A is a longitudinal sectional view showing a state before the overtube in Fig. 4A is connected to a driving portion body.
{Fig. 5B} Fig. 5B is a longitudinal sectional view showing a state after the overtube in Fig. 4A is connected to the driving portion body.
{Fig. 6} Fig. 6 is a block diagram for explaining the flow of information stored in the storage unit in the overtube in the medical manipulator system in Fig. 1.
{Fig. 7A} Fig. 7A is a partial longitudinal sectional view showing a state before connection to a driving portion body, showing a first modification of the overtube in Fig. 4A.
{Fig. 7B} Fig. 7B is a partial longitudinal sectional view showing a state after connection to a driving portion body, showing the first modification of the overtube in Fig. 4A.
{Fig. 8} Fig. 8 is a longitudinal sectional view showing a state before connection of a distal-end tubular portion and a proximal-end tubular portion, showing a second modification of the overtube in Fig. 4A.
{Fig. 9} Fig. 9 is a longitudinal sectional view showing state in which the distal-end tubular portion and the proximal-end tubular portion in the overtube in Fig. 8 are connected and attached to the driving portion body.
{Fig. 10} Fig. 10 is a longitudinal sectional view showing a third modification of the overtube in Fig. 4A.
{Fig. 11} Fig. 11 is a longitudinal sectional view showing a fourth modification of the overtube in Fig. 4A.
{Fig. 12} Fig. 12 is a longitudinal sectional view showing a fifth modification of the overtube in Fig. 4A.
{Fig. 13} Fig. 13 is a longitudinal sectional view showing a sixth modification of the overtube in Fig. 4A.
{Fig. 14} Fig. 14 is a longitudinal sectional view showing a seventh modification of the overtube in Fig. 4A.
{Fig. 15} Fig. 15 is a longitudinal sectional view showing an eighth modification of the overtube in Fig. 4A.
{Fig. 16} Fig. 16 is a longitudinal sectional view showing a ninth modification of the overtube in Fig. 4A.
{Fig. 17A} Fig. 17A is a partial perspective view for explaining a parallel connection form of connection sections of the distal-end tubular portion and the proximal-end tubular portion, showing a tenth modification of the overtube in Fig. 4A and Fig. 4B.
{Fig. 17B} Fig. 17B is a partial perspective view for explaining a connection form in which the connection sections of the distal-end tubular portion and the proximal-end tubular portion cross over, showing the tenth modification of the overtube in Fig. 4A and Fig. 4B.
{Fig. 18} Fig. 18 is a longitudinal sectional view showing an eleventh modification of the overtube in Fig. 4A.

### {Description of Embodiments}

An overtube 4 and a medical manipulator system 1 according to an embodiment of the present invention will be described below with reference to the drawings.

As shown in Fig. 1 and Fig. 2, the medical manipulator system 1 according to this embodiment is provided with: an endoscope 2 and two manipulators 3 that are inserted into the body of a patient P; the overtube 4 according to an embodiment of the present invention, which accommodates the endoscope 2 and the manipulators 3; an operating portion 5 that is operated by an operator A; a control portion 6 that controls each of the manipulators 3 on the basis of the operation of the operation portion 5; and a monitor 7.

As shown in Fig. 2 and Fig. 3, the manipulators 3 are each provided with: an insertion portion 8 that is inserted in the body of the patient P via a channel 13 in the overtube 4, described later; a movable portion 9 that is provided at the distal end of the insertion portion 8; and a driving portion 10 that is disposed at the proximal end of the insertion portion 8 and that drives the movable portion 9 by means of a motive-force transmitting member such as a wire or the like (not illustrated).

The movable portion 9 includes: a treatment portion 11 that is disposed at the extreme distal end and that acts on a diseased part in the body to perform treatment thereon; and a plurality of joints 12 that change the position and orientation of the distal end of the treatment portion 11.

As shown in Fig. 4A, the overtube 4 according to this embodiment, which is a tube formed of a material having flexibility, includes: a distal-end tubular portion 15 having two manipulator channels 13 through which the manipulators 3 are passed and a single endoscope channel 14 through which an endoscope 2 is passed; and a proximal-end tubular portion 16 having extension channels 13a that extend the two manipulator channels 13 from the proximal end of the distal-end tubular portion 15 towards the proximal-end side. A first housing 17 is fixed at the distal end of the distal-end tubular portion 15, a second housing 18 is fixed at the proximal end of the distal-end tubular portion 15, and a third housing 19 is fixed at the proximal end of the proximal-end tubular portion 16.

As shown in Fig. 4B, the first housing 17 is provided with distal-end openings 20 and 21 from which the distal ends of the two manipulators 3 inserted in the two manipulator channels 13 and the distal end of the endoscope 2 inserted in the endoscope channel 14 protrude, respectively.

The second housing 18 is provided with a proximal-end opening 22 into which the endoscope 2 is inserted.

As shown in Fig. 4C, the third housing 19 is provided with two proximal-end openings 23 into which the two manipulators 3 are inserted.

As shown in Fig. 4D, the two distal-end openings 20 from which the two manipulators 3 protrude may be provided so as to flank the distal end opening 21 of the endoscope channel 14, between the distal-end openings 20. Thus, the positional relationship of the distal-end openings 20 and 21 from which the manipulators 3 and the endoscope 2 protrude is different from the case in Fig. 4B, and accordingly, the protruding directions and orientations of the manipulators 3 in an image captured by the endoscope 2 change; however, by transferring information thereon to the control portion 6 from a memory circuit 24 according to this embodiment and changing the control parameters, improved operation become possible.

As shown in Fig. 4A, the third housing 19 is provided with a the memory circuit (storage unit) 24, which stores identification information and a usage count of the overtube 4 in a readable/writable manner, and a connection terminal 25 for reading out the identification information and usage count stored in the memory circuit 24 to the outside, or for writing a usage count from the outside, which are connected by wiring 26.

As shown in Fig. 2, the driving portion 10 of the manipulators 3 includes: a driving portion body 27 that includes motors; and manipulator-side driving portions 28 that are detachably provided on the driving portion body 27 and that, by being attached to the driving portion body 27, transfer the driving forces of the motors to the motive-force transmitting members in the insertion portions 8.

As shown in Fig. 5A and Fig. 5B, it is possible to detachably attach the third housing 19 of the overtube 4 to the driving portion body 27. The driving portion body 27 is provided with: a connection terminal 29 that contacts the connection terminal 25 provided in the third housing 19 when the third housing 19 is attached, thus being electrically connected thereto; and an input/output unit (external apparatus) 30 that reads and writes information in the memory circuit 24 via the connection terminal 25.

When the connection terminal 25 in the third housing 19 is connected to the connection terminal 29 in the driving portion body 27, electrical power is supplied to the memory circuit 24 via these connection terminals 25 and 29, so that the information in the memory circuit 24 can be read out via the connection terminals 25 and 29 and a reading unit (readout unit) 31 in the driving portion body 27.

As shown in Fig. 6, the input/output unit 30 is provided with the reading unit 31, which reads out the information in the memory circuit 24, and a writing unit 32 for writing usage count information in the memory circuit 24. Fig. 6 is a block diagram for explaining the flow of information stored in the memory circuit 24.

The identification information read out from the memory circuit 24 by the reading unit 31 is sent from the control portion 6 to the monitor 7, where the information is displayed, whereby the operator A can determine if the overtube 4 is appropriate.

A notifying unit 33 is connected to the control portion 6. The notifying unit 33 may be configured as any type of unit, for example, a monitor, a light-emitting part, a speaker or the like, and in the case where the overtube 4 exceeds a prescribed usage count, the operator A can be notified to that effect.

The usage count read out from the memory circuit 24 by the reading unit 31 is compared with a prescribed threshold in the control portion 6, and if, as a result of the comparison, the usage count exceeds the prescribed threshold, a notification to that effect is issued by the notifying unit 33.

At a certain timing, such as at system power up, the control portion 6 increments the usage count of the overtube 4, and can write it in the memory circuit 24 via the writing unit 32 in the input/output unit 30 and the connection terminals 25 and 29.

The operation of the overtube 4 and the medical manipulator system 1 according to this embodiment, configured in this way, will be described below.

To treat a diseased part in the body using the medical manipulator system 1 according to this embodiment, in the state in which the distal ends of the two manipulators 3 inserted in the extension channels 13a from the proximal-end openings 23 in the third housing 19 are disposed close to the distal-end openings 20 in the first housing 17 in the distal-end tubular portion 15 via the manipulator channels 13, and in which the distal end of the endoscope 2 inserted in the endoscope channel 14 from the proximal-end opening 22 provided in the second housing 18 is disposed close to the distal-end opening 21 in the first housing 17, the overtube 4 is inserted into the body of the patient P.

Then, in the state in which the distal end of the overtube 4 is disposed in contact with the diseased part in the body, the operator A causes the distal end of the endoscope 2 to protrude from the distal-end opening 21 in the endoscope channel 14 and causes the two movable portions 9 to protrude from the respective distal-end openings 20 in the manipulator channels 13. In this state, the third housing 19 of the proximal-end tubular portion 16 is fixed to the driving portion body 27, and the manipulator-side driving portion 28 is attached to the driving portion body 27.

As shown in Fig. 5B, when the third housing 19 is fixed to the driving portion body 27, the connection terminal 29 provided in the driving portion body 27 comes into contact with the connection terminal 25 provided in the third housing 19 to be electrically connected therewith, and when the power supply of the medical manipulator system 1 is turned on, power is supplied to the memory circuit 24. Accordingly, the identification information and usage count information of the overtube 4, which are stored in the memory circuit 24, are read by the reading unit 31. The read identification information and usage count information are sent to the control portion 6, the identification information is sent by the control portion 6 to the monitor 7 for display, and it is determined whether or not the usage count information exceeds the prescribed threshold.

By displaying the identification information on the monitor 7, the operator A can confirm whether or not the manipulator channel 13 in the overtube 4 is one that is suitable for the manipulator 3 to be used, without removing the overtube 4, which is inserted in the body, from the body.

If the length of the manipulator channel 13, the position of the distal-end opening 20, or the like are appropriate for the manipulator 3 to be used, it can be determined that manipulator channel 13 is a suitable one, whereas if the length of the manipulator channel 13 is large, if the length is extremely short, or if the position of the distal-end opening 20 differs from a desired position, etc., it can be determined that manipulator channel is not suitable for the manipulator 3. Therefore, in accordance with the displayed identification information, the operator A can change the manipulator 3 or can change the overtube 4, so as to perform appropriate treatment.

If, as a result of the determination in the control portion 6, the usage count exceeds the prescribed threshold, a notification to that effect is given by the notifying unit 33. If the usage count is less than or equal to the prescribed threshold, the usage count is incremented and is written in the memory circuit 24 via the writing unit 32 in the input/output unit 30 and the connection terminals 25 and 29.

Thus, with the overtube 4 and the manipulators 3 according to this embodiment, identification information of the manipulator channel 13 in the memory circuit 24 provided in the overtube 4 can be read out externally, and therefore, when the manipulator 3 is inserted into the manipulator channel 13 in the overtube 4 in the state in which the overtube 4 is inserted in the body, the appropriateness thereof can be determined without removing the overtube 4.

By configuring the memory circuit 24 so as to be readable and writable and by writing the usage count of the overtube 4 in the memory circuit 24, an advantage is afforded in that, during use, it is possible to issue a notification as to whether the usage count of the overtube 4 exceeds the prescribed threshold, it is possible to avoid the drawback wherein the overtube 4 is used in excess of a prescribed number of uses, and it is possible to appropriately manage reuse of the overtube 4.

The connection terminal 25 in the third housing 19 is connected to the connection terminal 29 in the driving portion body 27 so that the identification information and the usage count information are initially read out, and therefore, by displaying the identification information on the monitor 7, an advantage is afforded in that it is possible to confirm that the connection between the overtube 4 and the driving portion body 27 has been performed correctly.

In this embodiment, it has been described that the memory circuit 24 and the connection terminal 25 are provided in the overtube 4, that power is supplied by electrically connecting them to the connection terminal 29 provided in the driving portion body 27, and that the identification information etc. is read out from the memory circuit 24. Instead of this, as shown in Fig. 7A and Fig. 7B, a transmission/reception unit (wireless transmission unit) 35 that wirelessly transmits the information in the memory circuit 24 to an external wireless transmission/reception unit (external apparatus, readout unit) 34 and that receives information wirelessly transmitted from the external wireless transmission/reception unit 34 and writes it in the memory circuit 24 may be provided.

This transmission/reception unit 35 may be configured as a circuit that does not operate in the state in which it is disconnected from the driving portion body 27, as shown in Fig. 7A, and is capable of transmission and reception in conjunction with a circuit provided on the driving portion body 27 side, when the third housing 19 is connected to the driving portion body 27, as shown in Fig. 7B.

In this embodiment, the overtube 4 in which the distal-end tubular portion 15 and the proximal-end tubular portion 16 are formed as a single unit has been illustrated as an example; instead of this, however, as shown in Fig. 8, the distal-end tubular portion 15 and the proximal-end tubular portion 16 may be attached together in a detachable manner. Since the second housing 18 having the proximal-end opening 22 through with the endoscope 2 is inserted is disposed outside the body of the patient P, the proximal-end tubular portion 16, which is deposed closer to the proximal-end, is a part that is not inserted into the body of the patient P.

By separating the distal-end tubular portion 15, into which the endoscope 2 is inserted, and the proximal-end tubular portion 16, into which the endoscope 2 is not inserted, it is possible to facilitate the task of inserting the endoscope 2. In other words, in the case where the endoscope 2 is inserted into the body, in the state in which the endoscope 2 is made to pass through the endoscope channel 14 in the distal-end tubular portion 15, and the endoscope 2 is made to protrude by an adequate length from the distal-end opening 21 in the endoscope channel 14, only the endoscope 2 is inserted further into the body cavity.

In this state, the endoscope 2 is operated, and while checking the image acquired by the endoscope 2 on the monitor 7, the endoscope 2 is inserted farther into the body cavity of the patient P.

Once the distal end face of the endoscope 2 has advanced by a certain amount into the body, so that a prescribed site appears in the image, the distal-end tubular portion 15 disposed on the proximal-end side of the endoscope 2 is made to advance relative to the endoscope 2, so as to move along the endoscope 2, using the endoscope 2 as a guide. By repeating these operations, the endoscope 2 is inserted farther into the body cavity.

At this time, since the proximal-end tubular portion 16 is not connected to the proximal end of the distal-end tubular portion 15, the proximal-end tubular portion 16 does not obstruct the task of inserting the distal-end tubular portion 15, and thus the ease of insertion can be improved. Thereafter, in the state in which the distal-end tubular portion 15 is inserted until the distal end of the distal-end tubular portion 15 is disposed close to the distal end of the endoscope 2, preparations are completed by fitting the proximal-end of the tubular portion 16 into a hole 36 in the second housing 18, inserting the manipulator 3 via the proximal-end opening 23 in the third housing 19, and fixing the third housing 19 to the driving portion body 27, as well as by fixing the manipulator-side driving portion 28 to the driving portion body 27.

Thus, in the case where the distal-end tubular portion 15 and the proximal-end tubular portion 16 are detachably separated, it is necessary to individually manage the distal-end tubular portion 15 and the proximal-end tubular portion 16. To do so, as shown in Fig. 8, this overtube 4 is provided with a distal-end storage unit (storage unit) 37 and a proximal-end storage unit (storage unit) 38 that store identification information and information about usage counts etc. for the distal-end tubular portion 15 and the proximal-end tubular portion 16, respectively, and when the distal-end tubular portion 15 and the proximal-end tubular portion 16 are connected, the connection terminals 39 and 40 and the wires 26 that connect the two storage units 37 and 38 are provided in the distal-end tubular portion 15 an the proximal-end tubular portion 16, respectively.

In other words, the second housing 18 is provided with the distal-end storage unit 37, which stores the identification information and the usage count for the distal-end tubular portion 15, and is provided with the connection terminal 39, which is connected to the distal-end storage unit 37 and which is exposed, in the hole 36, at the inner circumferential surface that contacts the proximal-end tubular portion 16. On the other hand, the third housing 19 is provided with the proximal-end storage unit 38, which stores the identification information and usage count for the proximal-end tubular portion 16, the wire 26 connected to the proximal-end storage unit 38 extends along the longitudinal direction of the proximal-end tubular portion 16, and the connection terminal 40, which is connected to the connection terminal 39 when connected to the second housing 18, is provided so as to be exposed at the outer circumferential surface.

By doing so, as shown in Fig. 9, the distal-end of the proximal-end tubular portion 16 is fitted into the hole 36 in the second housing 18 of the distal-end tubular portion 15 so that the manipulator channel 13 and the extension channel 13a are connected, and the connection terminals 39 and 40 are connected with each other; and also, by attaching the third housing 19 to the driving portion body 27 and turning on the power supply, the information stored in the distal-end storage unit 37 is read out by the input/output unit 30 via the proximal-end storage unit 38. In addition, the information stored in the proximal-end storage unit 38 is read out directly. Furthermore, the usage count information from the control portion 6 is written into the distal-end storage unit 37 via the proximal-end storage unit 38, or is directly written into the proximal-end storage unit 38.

Accordingly, the operator can determine whether either one of the distal-end tubular portion 15 and the proximal-end tubular portion 16 is appropriate for the manipulator 3 to be used. In addition, in the case where the usage count of either one of the distal-end tubular portion 15 and the proximal-end tubular portion 16 exceeds a usage count that set therefor, a notification to that effect is given by the notifying unit 33, and therefore, it is possible to prevent a situation where the distal-end tubular portion 15 or the proximal-end tubular portion 16 exceeding the usage count is used.

In this case also, as shown in Fig. 10, the read-out of information from the storage units 37 and 38 or the writing of information into the storage units 37 and 38 may be performed wirelessly. In this case, the proximal-end storage unit 38 and the transmission/reception unit 35 in the proximal-end tubular portion 16 should be disposed close to the distal end of the proximal-end tubular portion 16, and the connection terminals 39 and 40 that are connected when the distal end of the proximal-end tubular portion 16 is fitted into the hole 36 in the second housing 18 should be provided in the proximal-end tubular portion 16 and the hole 36, respectively. Accordingly, the information for the proximal-end storage unit 38 is transmitted and received by the transmission/reception unit 35 directly, and the information for the distal-end storage unit 37 is transmitted and received by the transmission/reception unit 35 via the proximal-end storage unit 38.

As shown in Fig. 11, the information for the distal-end storage unit 37 may be transmitted and received by a transmission/reception unit (wireless transmission unit) 41 provided in the distal-end tubular portion 15, and the information for the proximal-end storage unit 38 may be transmitted and received by the transmission/reception unit 35 provided in the proximal-end tubular portion 16. At this time, it is preferable that transmission and reception from the two transmission/reception units 35 and 41 be enabled by connecting together the connection terminals 39 and 40 that are connected to the storage units 37 and 38, respectively.

As shown in Fig. 12, conversely to the case in Fig. 10, the transmission/reception unit 41 may be provided only in the distal-end tubular portion 15. Accordingly, the information for the proximal-end storage unit 38 is transmitted and received by the transmission/reception unit 41 via the distal-end storage unit 37, and the information for the distal-end storage unit 37 is transmitted and received by the transmission/reception unit 41 directly.

Even in the case where the distal-end tubular portion 15 and the proximal-end tubular portion 16 are detachably separated, when either one of them can be used only when paired with the other, as shown in Fig. 13, the storage unit 38 may be provided only in the proximal-end tubular portion 16, or alternatively, as shown in Fig. 14, the storage unit 37 may be provided only in the distal-end tubular portion 15. In addition, as shown in Fig. 15, the proximal-end section of the distal-end tubular portion 15 may be electrically connected by a wire with the control portion 6 via detachable connectors L1 and L2 so that information is transmitted and received, and in this case also, the transmission and reception may be performed wirelessly, as shown in Fig. 7 or Fig. 16.

As shown in Fig. 17A and Fig. 17B, in the case where, for example, there are two manipulator channels 13, and there are separate ends of the proximal-end tubular portions 16 that connect to the distal-end tubular portion 15 for each manipulator channel 13, so that it is possible to connect them to either of the manipulator channels 13, the configurations in Figs. 8 to 12 may be provided in each manipulator channel 13. Accordingly, it is also possible to easily confirm the connection states of the distal-end tubular portion 15 and the proximal-end tubular portions 16.

As shown in Fig. 18, a form in which the storage unit 37 is provided in the distal-end tubular portion 15 and performs transmission and reception wirelessly, a separate storage unit 38 is provided in the proximal-end tubular portion 16, and the storage unit 38 and the input/output unit 30 are electrically connected via the connection terminals 25 and 29 so as to transmit and receive information is also possible.

In this embodiment, the case where two manipulators 3 are provided has been illustrated as an example; however, the number of manipulator channels 13 may be 1 or may be 3 or more.

In the case where the medical manipulator system 1 is provided with a sterilization apparatus, the number of times that sterilization treatment has been performed on the overtube 4 by the sterilization apparatus may also be stored in the storage units 37 and 38.

Although the memory circuit 24 formed of an electrical circuit has been illustrated as an example of the storage units 37 and 38, instead of this, the storage units 37 and 38 may be configured of bar codes, and the reading unit 31 may be configured of a bar code reader. The storage units 37 and 38 may also be configured of RFIDs.

### {Reference Signs List}

1 medical manipulator system
3 manipulator
4 overtube
5 operating portion
6 control portion
9 movable portion
10 driving portion
13 manipulator channel
13a extension channel
14 endoscope channel
15 distal-end tubular portion
16 proximal-end tubular portion
24 memory circuit (storage unit)
25, 29, 39, 40 connection terminal
30 input/output unit (external apparatus)
31 reading unit (readout unit)
32 writing unit
33 notifying unit
34 wireless transmission/reception unit (external apparatus, readout unit)
35, 41 transmission/reception unit (wireless transmission unit)
37 distal-end storage unit (storage unit)
38 proximal-end storage unit (storage unit)
A operator

## Claims

1. An overtube comprising:
a manipulator channel through which is passed a long manipulator in which a movable portion disposed at a distal end is driven by a driving portion disposed at a proximal end; and
a storage unit that stores identification information for the manipulator channel in a readable manner from outside.

2. An overtube according to claim 1, further comprising an endoscope channel through which an endoscope is passed.

3. An overtube according to claim 2, further comprising:
a distal-end tubular portion in which the manipulator channel and the endoscope channel are provided; and
a proximal-end tubular portion that is detachably provided at the proximal end of the distal-end tubular portion and that is provided with an extension channel that extends the manipulator channel at the proximal-end side,
wherein the storage unit is provided in at least one of the distal-end tubular portion or the proximal-end tubular portion.

4. An overtube according to claim 3, wherein the storage unit is formed of an electrical circuit provided in either one of the distal-end tubular portion or the proximal-end tubular portion; and
a proximal-end section of the distal-end tubular portion or a proximal-end section of the proximal-end tubular portion is provided with a connection terminal for an external apparatus to read out the identification information, when the distal-end tubular portion or the proximal-end tubular portion having said proximal-end section is connected to the external apparatus.

5. An overtube according to claim 3, wherein the storage unit is formed of an electrical circuit provided in either one of the distal-end tubular portion or the proximal-end tubular portion; and
a wireless transmission unit that can wirelessly read out the identification information from an external apparatus when the distal-end tubular portion and the proximal-end tubular portion are connected is connected to the storage unit.

6. An overtube according to claim 3, wherein the storage unit is provided with a distal-end storage unit that is provided in the distal-end tubular portion and that stores identification information of the manipulator channel, and a proximal-end storage unit that is provided in the proximal-end tubular portion and that stores identification information of the extension channel.

7. An overtube according to claim 6, wherein the distal-end storage unit and the proximal-end storage unit are formed of electrical circuits that are connected when the distal-end tubular portion and the proximal-end tubular portion are connected; and
a connection terminal for an external apparatus to read out the identification information when the proximal-end tubular portion is connected to the external apparatus is provided in the proximal-end section of the proximal-end tubular portion.

8. An overtube according to claim 6, wherein the distal-end storage unit and the proximal-end storage unit are formed of electrical circuits; and a wireless transmission unit that can wirelessly read out the identification information from an external apparatus when the distal-end tubular portion and the proximal-end tubular portion are connected is connected to at least one of the distal-end storage unit or the proximal-end storage unit.

9. An overtube according to one of claims 4 to 8, wherein the storage unit stores usage count information in a readable and writable manner.

10. An overtube according to one of claims 1 to 9, wherein a plurality of the manipulator channels are provided.

11. An overtube according to claim 7 or 8, wherein the distal-end tubular portion is provided with a plurality of the manipulator channels;
the proximal-end tubular portion is provided with, in the same number as the number of manipulator channels, a plurality of the extension channels that can extend the manipulator channels, in arbitrary combinations;
the distal-end storage unit is provided in each of the manipulator channels; and
the proximal-end storage unit is provided in each of the extension channels.

12. A medical manipulator system comprising:
an overtube according to one of claims 1 to 11;
the manipulator, which is inserted into the manipulator channel in the overtube;
an operating portion that is operated by an operator;
a control portion that controls the driving portion on the basis of an operation input that is input via the operation portion; and
a readout unit that reads out information stored in the storage unit.

13. A medical manipulator system comprising:
an overtube according to claim 9;
the manipulator, which is inserted into the manipulator channel in the overtube;
an operating portion that is operated by an operator;
a control portion that controls the driving portion on the basis of an operation input that is input via the operating portion;
a readout unit that reads out information stored in the storage unit;
a writing unit that writes a usage count of the overtube into the storage unit; and
a notifying unit that, when the usage count read out from the storage unit by the readout unit exceeds a prescribed count, issues a notification to that effect.
